# EUROPÄISCHE PATENTSCHRIFT

(11) **EP 1 616 848 B1**
(45) Veröffentlichungstag und Bekanntmachung des Hinweises auf die Patenterteilung: **22.01.2014**
(21) Anmeldenummer: 05103618.4
(22) Anmeldetag: 02.05.2005
(51) Int. Cl.: C07C 29/04, C07C 31/12

(54) **Verfahren zur Herstellung von tert.-Butanol aus Isobuten-haltigen Kohlenwasserstoffgemischen**
Process for the preparation of tert.-butanol from isobutene containing hydrocarbon mixtures
Procédé de préparation de tert.-butanol à partir de mélanges d'hydrocarbures contenant de l'isobutène

(30) Priorität: 26.06.2004 DE 102004030943
(43) Veröffentlichungstag der Anmeldung: 18.01.2006
(73) Patentinhaber: Evonik Degussa GmbH, 45128 Essen (DE)
(72) Erfinder: Santiago Fernandez, Silvia, 46049, Oberhausen (DE); Sauer, Jörg Dr., 48249, Dülmen (DE); Stochniol, Guido Dr., 45721, Haltern am See (DE); Maschmeyer, Dietrich Dr., 45657, Recklinghausen (DE); Büschken, Wilfried Dr., 45721, Haltern am See (DE); Wiese, Klaus-Diether Dr., 45721, Haltern am See (DE)
(74) Vertreter: Hirsch, Hans-Ludwig

(56) Entgegenhaltungen:
- EP-A- 1 431 264
- EP-A- 1 508 558

## Beschreibung

Die Erfindung betrifft ein Verfahren zur Herstellung von tert.-Butanol durch Anlagerung von Wasser an Isobuten in Gegenwart eines sauren Ionenaustauschers in einer Reaktorkaskade, indem zumindest in einen Reaktor im Wechsel Gemische mit unterschiedlichen tert.-Butanol- und Wassergehalt gefahren werden.

Tert.-Butanol (TBA) ist ein wichtiges, großtechnisch hergestelltes Produkt und wird als Lösungsmittel und als Zwischenprodukt für die Herstellung von Methacrylsäuremethylester verwendet. Es ist Vorstufe für die Herstellung von Peroxiden, wie Peroxiketale, Perester oder Dialkylperoxide, mit mindestens einer tertiären Butylgruppe. Diese Verbindungen werden als Oxidationsmittel und als Starter für Radikalreaktionen, wie beispielsweise Olefinpolymerisation oder Vernetzung von Kunststoffen, eingesetzt. Als Zwischenstufe dient tert.-Butanol zur Gewinnung von reinem Isobuten aus Isobutengemischen. Darüber hinaus ist es ein Reagenz zur Einführung von tertiären Butylgruppen. Seine Alkalisalze sind starke Basen, die in vielen Synthesen Verwendung finden.

Tertiäres Butanol kann durch sauer katalysierte Anlagerung von Wasser an Isobuten erhalten werden. Technische Isobutengemische enthalten häufig auch andere Olefine, wie z. B. 2-Butene. Werden diese Edukte eingesetzt, so wenden technische Verfahren Bedingungen an, bei denen fast ausschließlich das Isobuten, nicht aber die anderen Olefine hydratisiert werden und Nebenreaktionen, wie Homo- oder Heterooligomerisierung der Olefine, nahezu vollständig unterdrückt werden. Solche Prozesse arbeiten gewöhnlich in der flüssigen Phase und können in zwei Gruppen unterteilt werden: a) Verfahren, bei denen die Umsetzung in einer wasserhaltigen Katalysatorlösung erfolgt und b) heterogene katalytische Prozesse, bei denen feste, in der Reaktionsphase unlösliche Katalysatoren eingesetzt werden.

Die Hydratisierung von Isobuten zu tert.-Butanol mit Hilfe fester, weder in den Edukten noch in den Produkten löslicher, saurer Katalysatoren hat den Vorteil, dass das Reaktionsgemisch säurefrei ist und ohne Verluste durch Rückspaltung oder durch andere Nebenreaktionen zu tert.-Butanol aufgearbeitet werden kann. Die Reaktion läuft an der Oberfläche des Katalysators ab. Damit eine Reaktion zustande kommt, müssen beide Reaktionspartner zur gleichen Zeit an der aktiven Stelle des Katalysators vorhanden sein. Dies wird dadurch erschwert, das Wasser und Isobuten bzw. ein Isobuten-haltiges Kohlenwasserstoffgemisch nicht miteinander mischbar sind. Um akzeptable Umsätze zu erhalten, werden Solventien verwendet, die ein homogenes Gemisch aus Wasser und Isobuten-Einsatzgemisch ermöglichen.

In DE 30 31 702 A1 wird Methanol für diesen Zweck als Lösungsmittel sowohl für Wasser als auch für Isobuten bzw. für ein Isobuten-haltiges Kohlenwasserstoffgemisch beschrieben. Als Produkte werden tert.-Butanol und Methyl-tert.-butylether nebeneinander erhalten.

In EP 0 010 993 A1 werden aliphatische Carbonsäuren mit 1 bis 6 C-Atomen als Lösungsmittel für beide Edukte eingesetzt. Dabei entstehen als Nebenprodukte die tertiären Butylester dieser Säuren. Diese müssen zu tert.-Butanol und Carbonsäuren hydrolisiert werden.

In DE 30 31 702 A1 werden Sulfolane und in US 4,327,231 mehrwertige Alkohole des Neo-Typs, wie beispielsweise Neopentylglykol, eingesetzt. Diese Lösungsmittel müssen vom tert.-Butanol abgetrennt werden. Darüber hinaus besteht die Gefahr, dass das verwendete Lösungsmittel im Dauerbetrieb einer solchen Anlage zersetzt wird.

Um diese Nachteile zu vermeiden, wird in einigen Verfahren das Zielprodukt, TBA, als Lösevermittler eingesetzt. Solche Verfahren sind beispielsweise in WO 99/33775, DE 30 25 262 oder DE 102 59 413.9 beschrieben. Dabei wird ein Gemisch aus einer Kohlenwasserstofffraktion, die Isobuten enthält, TBA und Wasser in einer Reaktorkaskade an sauren im Festbett angeordneten Katalysatoren umgesetzt. Der erste Reaktor wird meistens in Schlaufenfahrweise und die anderen im geraden Durchgang betrieben. Vor jedem weiteren Reaktor kann Reaktionswasser zudosiert werden. Der Austrag des letzten Reaktors wird destillativ in ein Kohlenwasserstoffgemisch mit dem nicht umgesetzten Isobuten und Roh-TBA getrennt. Ein Teil des Roh-TBA wird in den ersten Reaktor zurückgeführt. Der andere Teil kann als solcher genutzt werden oder auf TBA und/oder TBA/Wasser-Azeotrop aufgearbeitet werden.

Bei diesen Verfahren nimmt von Reaktor zu Reaktor aufgrund des Reaktionsfortschritts der TBA-Gehalt zu und der Isobutengehalt ab. Die Zusammensetzung des Reaktionsgemisch nähert sich dabei mit abnehmender Geschwindigkeit dem thermodynamischen Gleichgewicht zwischen Wasser, Isobuten und TBA, sodass kein vollständiger Umsatz erreicht werden kann. Dabei können bei langen Reaktionszeiten Umsätze von etwa 93 % erreicht werden. Ist jedoch eine höhere Raum-Zeit-Ausbeute erwünscht, ist es zweckmäßig den Umsatz zu begrenzen. Beispielsweise kann es vorteilhaft sein, ausgehend von technischen Isobutenströmen, beispielsweise von Raffinat I, den Umsatz auf 80 bis 85 % zu begrenzen.

In der noch nicht offengelegten Anmeldung DE 103 38 581 wird die Herstellung von tert.-Butanol durch Umsetzung von Isobuten-haltigen Kohlenwasserstoffgemischen mit Wasser an sauren festen Katalysatoren in mehreren Reaktoren beschrieben, wobei zur Erhöhung des Umsatzes vor dem letzten Reaktor ein Teil des im Reaktionsgemisch vorhandenen TBA abgetrennt wird. Trotz dieser Verbesserung steigt der Gesamt-Isobuten-Umsatz bei etwa gleicher Raum-Zeit-Ausbeute nur um 5 bis 10 % auf etwa 90 %.

In der noch nicht offengelegten Anmeldung DE 102 60 991 wird ein Verfahren zur Herstellung von TBA beschrieben, bei dem eine relativ aufwändige Reaktivdestillation eingesetzt wird und bei nahezu vollständigem Isobuten-Umsatz aus Isobuten auch Hochsieder entstehen.

EP 1 508 558 beschreibt ein Verfahren zur Herstellung von tert.-Butanol (TBA) durch Umsetzung von Isobuten-haltigen Kohlenwasserstoffgemischen mit Wasser an sauren festen Katalysatoren in mehreren Reaktoren, wobei zur Erhöhung des Umsatzes vor dem letzten Reaktor ein Teil des im Reaktionsgemisch vorhandenen TBA abgetrennt wird.

In EP 1 431 264 wird ein Verfahren zur Herstellung von tert.-Butanol durch Anlagerung von Wasser an Isobuten in Gegenwart eines saueren Ionenaustauschers beschrieben.

Da die bekannten Verfahren, die feste, saure und im Reaktionsmedium unlösliche Stoffe als Katalysator verwenden, zwar eine einfache Aufarbeitung bieten, jedoch hinsichtlich Raum-Zeit-Ausbeute und/oder Ausbeute und/oder Selektivität nicht befriedigen, bestand die Aufgabe der vorliegenden Erfindung darin, ein Verfahren bereitzustellen, welches sich durch eine hohe Raum-Zeit-Ausbeute und eine hohe Selektivität bei hohem Isobuten-Umsatz auszeichnet.

Es wurde überraschend gefunden, dass eine hohe Raum-Zeit-Ausbeute in einem mit festen sauren Stoffen gefüllten Reaktor bei der Umsetzung von Isobuten mit Wasser zu Butanol bei guter Selektivität erzielt werden kann, wenn der Reaktor abwechselnd mit einem Gemisch aus Isobuten-haltigen Kohlenwasserstoffgemisch, TBA und Wasser und einem Wasser- und TBA-ärmeren, Isobuten-haltigen Kohlenwasserstoff-Gemisch beschickt wird.

Gegenstand der vorliegenden Erfindung ist deshalb ein Verfahren zur Herstellung von tert.-Butanol (TBA) durch Umsetzung eines Isobuten-haltigen Kohlenwasserstoffgemisches mit Wasser an einem festen sauren Katalysator in einer Reaktorkaskade (die zumindest zwei oder mehr in Reihe geschaltete Reaktoren aufweist), welches dadurch gekennzeichnet ist, dass mindestens ein Reaktor wechselweise mit zwei verschiedenen Isobuten-haltigen Kohlenwasserstoffgemischen beschickt wird, wobei eines der Gemische sowohl einen höheren tert.-Butanol-Gehalt als auch einen höheren Wassergehalt als das andere Gemisch aufweist.

Außerdem ist Gegenstand der vorliegenden Erfindung ein Tert.-Butanol aufweisendes Gemisch, erhältlich durch das erfindungsgemäße Verfahren, insbesondere durch ein erfindungsgemäßes Verfahren wie in den Ansprüchen beansprucht.

Die Reaktivität eines sauren Ionenaustauscherharzes bei der Umsetzung eines Olefins ist u. a. von der Größe seiner Hydrathülle abhängig ist. Trockenes oder nahezu trockenes Ionenaustauscherharz ist hochreaktiv, aber wenig selektiv. Es katalysiert nicht nur die Addition von Wasser an Isobuten zu TBA, sondern auch die Oligomerisation von Isobuten und von anderen im Reaktionsgemisch vorliegenden Olefinen sowie die Umsetzung von linearen Butenen zu 2-Butanol. Mit Wasser gesättigtes saures Ionenaustauscherharz dagegen ist so wenig reaktiv, dass es die Addition von Wasser an Isobuten fast gar nicht katalysiert. Die Größe der Hydrathülle und damit die katalytische Aktivität des Ionenaustauscherharzes ist u. a. von dem Wassergehalt im umfließenden Medium abhängig. Deshalb wird die Umsetzung von Isobuten-haltigen Kohlenwasserstoffgemischen mit Wasser in bestimmten Konzentrationsbereichen durchgeführt. Beispielsweise wird in DE 102 59 413.9 dargelegt, dass es besonders günstig ist, ein Gemisch, das aus einem Isobuten-haltigen Kohlenwasserstoffgemisch, TBA und Wasser besteht, zu TBA umzusetzen, wenn das Dreikomponentengemisch 30 bis 80 % der maximalen durch die Löslichkeit von Wasser im Gemisch von TBA und dem Isobuten-haltigen Kohlenwasserstoffgemisch möglichen Wassermenge enthält

Es war deshalb sehr überraschend, dass bei wechselweisem Fahren eines Reaktors mit zwei unterschiedlichen Einsatzstoffgemischen, wobei zumindest eines der Einsatzstoffgemische eine Zusammensetzung außerhalb des im Stand der Technik angegebenen optimalen Bereichs aufweist, ein hoher Umsatz erreicht wird. Vollkommen überraschend war, dass trotz Einspeisung eines Wasser- und TBA-armen bzw. praktisch Wasser- und TBA-freien Kohlenwasserstoffgemisches keine Abnahme der Selektivität der TBA-Bildung (betrachtet insgesamt über beide Fahrzustände) festzustellen war.

In den bekannten technischen Verfahren zur Herstellung von TBA sinkt aufgrund des Reaktionsfortschritts im Reaktionsgemisch von Reaktor zu Reaktor die Konzentration an Isobuten und die Konzentration an TBA nimmt zu. Mit von Reaktor zu Reaktor abnehmender Geschwindigkeit nähert sich die Zusammensetzung des Reaktionsgemisches dem thermodynamischen Gleichgewicht zwischen Isobuten, Wasser und TBA.

Um den Isobuten-Umsatz zu TBA zu erhöhen, wird im erfindungsgemäßen Verfahren aus dem den letzten Reaktor verlassenden Reaktionsgemisch die Kohlenwasserstofffraktion mit dem nicht umgesetzten Isobuten abgetrennt und diese Fraktion ganz oder teilweise in mindestens einen, bevorzugt den letzten Reaktor zurückgefahren, mit der Maßgabe, dass dieser Reaktor wechselweise mit dem Reaktionsgemisch des vorherigen Reaktors und mit gegebenenfalls zusätzlichem Wasser oder mit dem zurückgeführten Isobuten-haltigen Kohlenwasserstoffgemisch als Eduktgemisch beschickt wird. Da der rückgeführte Isobuten-haltige Strom praktisch kein TBA enthält, ist er weit vom Gleichgewicht zwischen TBA, Isobuten und Wasser entfernt. Es kann also ein großer Teil des Isobutens zu TBA umgesetzt werden. Als Reaktionswasser dient ein Teil der Hydrathülle des sauren Katalysators. Somit wird durch die Umsetzung der Isobuten-haltigen Kohlenwasserstofffraktion der Katalysator teilweise entwässert, wodurch seine Aktivität steigt. Nach Umschaltung und Beschickung des Reaktors mit einem Gemisch aus Wasser und dem Reaktionsaustrag des vorherigen Reaktors ist die Reaktionsgeschwindigkeit für die Umsetzung von Isobuten mit Wasser zu TBA zunächst größer und fällt in Folge der Wasseraufnahme des Katalysators auf den üblichen Wert ab. Über beide Fahrzustände gemittelt, ergibt sich für diesen Reaktor eine höhere Raum-Zeit-Ausbeute als bei herkömmlicher Fahrweise.

Der Vorteil der vorliegenden Erfindung liegt darin, dass bei gleich bleibender Selektivität der Umsatz an Isobuten und somit die Ausbeute an TBA erhöht wird.

Nachfolgend wird das erfindungsgemäße Verfahren beispielhaft beschrieben, ohne dass die Erfindung, deren Schutzbereich sich aus den Ansprüchen und der Beschreibung ergibt, darauf beschränkt sein soll. Auch die Ansprüche selbst gehören zum Offenbarungsgehalt der vorliegenden Erfindung. Sind im nachfolgenden Text Bereiche bzw. Vorzugsbereiche angegeben, so sollen auch alle in diesen Bereichen liegenden, theoretisch möglichen Teilbereiche zum Offenbarungsgehalt der vorliegenden Erfindung gehören, ohne dass diese aus Gründen der besseren Übersichtlichkeit explizit genannt worden sind.

Das erfindungsgemäße Verfahren zur Herstellung von TBA basiert auf den im Stand der Technik z. B. in WO 99/33775, DE 30 25 262 oder DE 102 59 413 beschriebenen Verfahren zur Herstellung von TBA, wobei im erfindungsgemäßen Verfahren zumindest ein Reaktor wechselweise mit zwei verschiedenen Isobuten-haltigen Kohlenwasserstoffgemischen, die unterschiedliche Gehalte an Wasser und TBA aufweisen, beschickt wird. Die im angegebenen Stand der Technik genannten Verfahrensparameter für die Herstellung von TBA sind deshalb auch ohne explizite Nennung Bestandteil der vorliegenden Beschreibung.

Das erfindungsgemäße Verfahren zur Herstellung von tert.-Butanol (TBA) durch Umsetzung eines Isobuten-haltigen Kohlenwasserstoffgemisches mit Wasser an einem festen sauren Katalysator in einer Reaktorkaskade, die zumindest zwei Reaktoren aufweist, zeichnet sich dadurch aus, dass mindestens ein Reaktor wechselweise mit zwei verschiedenen Isobuten-haltigen Kohlenwasserstoffgemischen beschickt wird, wobei eines der Gemische (I) sowohl einen höheren tert.-Butanol-Gehalt als auch einen höheren Wassergehalt als das andere Gemisch (II) aufweist. Vorzugsweise ist der Reaktor, der wechselweise mit zwei verschiedenen Isobuten-haltigen Kohlenwasserstoffgemischen beschickt wird, wobei eines der Gemische sowohl einen höheren tert.-Butanol-Gehalt als auch einen höheren Wassergehalt als das andere Gemisch aufweist, der letzte Reaktor der Reaktorkaskade oder eine letzte Reaktoreinheit aus zwei oder mehreren parallel geschalteten Reaktoren der Reaktorkaskade.

In den ersten Reaktor der Reaktorkaskade wird vorzugsweise ein Kohlenwasserstoffgemisch gefahren, welches einen Gehalt an Isobuten von größer 25 Massen-% aufweist. Als Isobuten-haltiges Eduktgemisch, das dem ersten Reaktor der Reaktorkaskade zugeführt wird, kann in dem erfindungsgemäßen Verfahren ein Isobuten-haltiges Kohlenwasserstoffgemisch, oder optional auch reines Isobuten eingesetzt werden. Vorzugsweise enthält das Isobuten-haltige Kohlenwasserstoffgemisch keine Acetylenderivate und/oder weniger als 5000 ppm an Dienen und/oder keine weiteren Olefine mit einer oder zwei Verzweigung(en) an der olefinischen Doppelbindung. Besonders bevorzugt weist der Isobuten-haltige Ausgangsstoff einen Gehalt an Isobuten von 25 bis 100 Massen-%, vorzugsweise von 30 bis 99 Massen-% und ganz besonders bevorzugt von 30 bis 80 Massen-% auf.

Technische Gemische, die Isobuten enthalten, sind beispielsweise Leichtbenzinftaktionen aus Raffinerien, C₄-Fraktionen aus FCC-Einheiten oder Steamcrackern, Gemische aus Fischer-Tropsch-Synthesen, Gemische aus der Dehydrierung von Butanen, Gemische aus Skelettisomerisierung linearer Butene oder Gemische, entstanden durch Metathese von Olefinen oder aus anderen technischen Prozessen.

Diese Gemische können ggf. nach Entfernung der mehrfach ungesättigten Verbindungen im erfindungsgemäßen Verfahren eingesetzt werden. Beispielsweise kann die Gewinnung eines geeigneten Isobutengemisches aus der C₄-Fraktion eines Steamcrackers durch Extraktion des Butadiens oder durch dessen Selektivhydrierung zu linearen Butenen erfolgen. Dieser Einsatzstoff (Raffinat I bzw. selektivhydriertes Crack-C₄) besteht aus n-Butan, Isobutan, den drei linearen Butenen und Isobuten und ist ein bevorzugtes Edukt für das erfindungsgemäße Verfahren. Der Isobuten-Gehalt im bevorzugt eingesetzten Raffinat I liegt typischerweise im Bereich von 30 bis 60 %.

Optional kann Raffinat I, hydriertes Crack-C₄ oder ein ähnlich zusammengesetztes Kohlenwasserstoffgemisch in einer Reaktivkolonne hydroisomerisiert werden. Dadurch kann ein Gemisch aus Isobuten, (und ggf. 1-Buten) und Isobutan gewonnen werden, welches dann als Rohstoff in der erfindungsgemäßen TBA-Synthese als Eduktgemisch (Strom (2)) eingesetzt werden kann.

Die Konzentration des Isobutens im Kohlenwasserstoffgemisch kann wie bereits gesagt in einem weiten Bereich variieren. Es ist jedoch wegen der Wirtschaftlichkeit des Verfahrens bevorzugt, Kohlenwasserstoffgemische mit einer Isobutenkonzentration größer 30 Massen-%, vorzugsweise größer 40 Massen-% als Eduktgemisch einzusetzen.

Als Katalysator wird bevorzugt ein saurer Ionenaustauscher, der weder im Einsatzstoffgemisch noch im Produktgemisch löslich ist, eingesetzt. Der Katalysator darf unter Reaktionsbedingungen weder durch Hydrolyse noch durch andere Reaktionen saure Stoffe an das Produktgemisch abgeben, weil dies zu Ausbeuteverlusten bei der Aufarbeitung des Reaktionsgemischs führen würde.

Für die Aktivität der geeigneten Katalysatoren gilt, dass sie unter Reaktionsbedingungen die Hydratisierung von Isobuten bewirkt, jedoch kaum die von nicht verzweigten Olefinen. Weiterhin dürfen die Katalysatoren die Oligomerisierung von Olefinen kaum katalysieren.

Eine geeignete Gruppe von Katalysatoren sind feste Ionenaustauscherharze mit Sulfonsäuregruppen. Besonders geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfongruppen verwendet. Die Harze können gelförmig, makroporös oder schwammförmig hergestellt werden. Geeignete Harze des Styrol-Divinylbenzol-Typs werden u. a. unter folgenden Handelsnamen verkauft: Duolite® C-20, Duolite® C-26, Amberlyst® 15Wet, Amberlyst® 35Wet, Amberlite® IR-120, Amberlite® 200C, Dowex® 50, Lewatit® K2621, Lewatit® K2629 oder Lewatit® VP OC 1505.

Die Eigenschaften dieser Harze, insbesondere spezifische Oberfläche, Porosität, Stabilität, Quellung bzw. Schrumpfung und Austauschkapazität, sind variabel und können durch den Herstellprozess variiert werden.

Im erfindungsgemäßen Verfahren werden die Ionenaustauscherharze bevorzugt in ihrer H-Form verwendet. Die Ionenaustauscherkapazität liegt zwischen 2 bis 7, insbesondere 3 bis 6 eq/kg (bezogen auf feuchtes handelsübliches Harz). Es werden vorzugsweise makroporöse Harze eingesetzt, wie beispielsweise Amberlyst® 15Wet, Amberlyst® 35Wet, Lewatit® K2621, Lewatit® K2629 oder Lewatit® VP OC 1505. Die mittlere Korngröße des Harzes kann bevorzugt von 0,5 bis 2 mm betragen. Die Korngrößenverteilung kann eng oder weit gewählt werden. Bevorzugt werden Ionenaustauscherharze mit sehr einheitlicher Korngröße (monodisperse Harze) eingesetzt. Bei der Verwendung mehrerer Reaktoren können diese mit Harz gleicher oder unterschiedlicher Korngröße (bzw. Korngrößenverteilung) gefüllt sein.

Optional können die Ionenaustauscherharze nicht nur als Kugeln, sondern auch als Formkörper, wie beispielsweise Zylinder, Ringe oder Kugeln, oder auf Formkörper aufpolymerisiert eingesetzt werden.

Es kann vorteilhaft sein, in Reaktoren, die mit hohen Lineargeschwindigkeiten durchströmt werden, zur Verringerung des Differenzdruckes ein Ionenaustauscherharz mit einem größeren Korn einzusetzen, und in Reaktoren, die mit einer geringen Lineargeschwindigkeit durchströmt werden, zur Erzielung des optimalen Umsatzes ein Ionenaustauscherhan mit kleinerem Korn einzusetzen. Bevorzugt werden in den Reaktoren des erfindungsgemäßen Verfahrens, besonders bevorzugt in solchen, die mit einer Lineargeschwindigkeit von 1 bis 60 m/h betrieben werden, Ionenaustauscherharze eingesetzt, die eine mittlere Korngröße von 0,1 bis 5 mm, vorzugsweise von 0,25 bis 2 mm und ganz besonders bevorzugt von 0,75 bis 1 mm aufweisen. Bei Lineargeschwindigkeiten von 10 bis 60 m/h werden vorzugsweise mittlere Korngrößen von 0,5 bis 5 mm eingesetzt. Bei Lineargeschwindigkeiten von 1 bis 25 m/h werden vorzugsweise mittlere Korngrößen von 0,1 bis 1 mm eingesetzt. Ein Ionenaustauscherharz mit einer mittleren Korngröße, die im Bereich von 0,70 bis 1 mm liegt, ist z. B. Amberlyst® 35Wet der Firma Rohm und Haas.

Um zu verhindern, dass das Harz während des Betriebs saure Gruppen abspaltet und somit Störungen im Aufarbeitungsteil des Verfahrens verursachen könnte und um eine hohe Aktivität des Katalysators über einen langen Zeitraum beizubehalten, kann das Ionenaustauscherharz vorbehandelt werden, beispielsweise durch Spülen mit Wasser, TBA oder TBA/Wasser-Gemischen vorzugsweise in einem Temperaturbereich von 40 bis 120 °C.

In den Reaktoren der Reaktorkaskade können jeweils die gleichen Katalysatoren oder Katalysatormischungen oder unterschiedliche Katalysatoren oder Katalysatormischungen eingesetzt werden. Vorzugsweise sind alle Reaktoren der Reaktorkaskade mit den gleichen Katalysatoren bzw. Katalysatormischungen ausgerüstet.

Das Kohlenwasserstoffgemisch (II) mit dem geringeren tert.-Butanol- und Wassergehalt, welches dem im Wechsel betriebenen Reaktor zugeführt wird, enthält vorzugsweise kleiner oder gleich 1 Massen-% tert.-Butanol. Bevorzugt beträgt der Gehalt an TBA in diesem Gemisch kleiner oder gleich 0,5 Massen-%, ganz besonders kleiner oder gleich 0,1 Massen-%. Die Wasserkonzentration dieses an Wasser ärmeren Gemisches ist maximal 10 % größer, gleich oder geringer als die maximale Lösungskonzentration (Löslichkeit) im entsprechenden Gemisch. Vorzugsweise wird im erfindungsgemäßen Verfahren eine homogene Lösung als Gemisch eingesetzt, dass heißt, das die Wasserkonzentration im eingesetzten Gemisch (II) gleich oder kleiner als die maximal mögliche Lösungskonzentration ist. Es kann vorteilhaft sein, wenn das Kohlenwasserstoffgemisch (II) mit dem geringeren tert.-Butanol-Gehalt einen Gehalt an Isobuten von 1 bis 30 Massen-% aufweist. Als an Wasser ärmeres Gemisch (II) wird vorzugsweise ein Gemisch verwendet, welches bei der destillativen Trennung des Reaktoraustrags des oder der wechselseitig betriebenen Reaktoren als Kopfprodukt bei der Abtrennung des TBA aus dem Reaktoraustrag anfällt, wobei bei der Destillation TBA bzw. Roh-TBA als Sumpfprodukt erhalten wird.

Das an Wasser reichere Gemisch (I), das im Wechsel in den Reaktor eingespeist wird, enthält Wasser, TBA und ein Isobuten-haltiges Kohlenwasserstoffgemisch. Das Gemisch (I) kann im erfindungsgemäßen Verfahren homogen oder heterogen vorliegen. Bei einem heterogenen Gemisch liegt ein Teil des Wassers in einer zweiten wässrigen Phase vor. Da bei der Beschickung des Reaktors mit einem an Wasser armen Isobuten-haltigen Kohlenwasserstoffstrom der Katalysator einen Teil seiner Hydrathülle verliert, muss mit dem an Wasser reicheren Eduktgemisch (I) mehr Wasser in den Reaktor gebracht werden als bei der üblichen Fahrweise, damit genügend Wasser zum Aufbau der Hydrathülle und als Reaktionswasser zur Verfügung steht Daher kann es je nach Verhältnis des Kohlenwasserstoffgemischs zu TBA und den anderen Betriebsparametern zweckmäßig sein, ein heterogenes Gemisch einzuspeisen. Die optimale Wasserkonzentration im Edukt kann durch einfache Vorversuche gefunden werden. Vorzugsweise ist die Konzentration an Wasser im Gemisch (I) um zumindest 11 %, vorzugsweise um zumindest 15 % und besonders bevorzugt um zumindest 25 % größer, als die maximale Lösungskonzentration (Löslichkeit) im entsprechenden Gemisch. Ganz besonders bevorzugt beträgt die Konzentration an Wasser im Gemisch (I) von 125 bis 175 % der maximalen Lösungskonzentration (Löslichkeit) im entsprechenden Gemisch. Als an Wasser reicheres Gemisch (I) kann z. B. der Reaktoraustrag eines in der Reaktorkaskade vorgeschalteten Reaktors eingesetzt werden, wobei diesem vorzugsweise ebenfalls zusätzliches Wasser zugegeben werden kann.

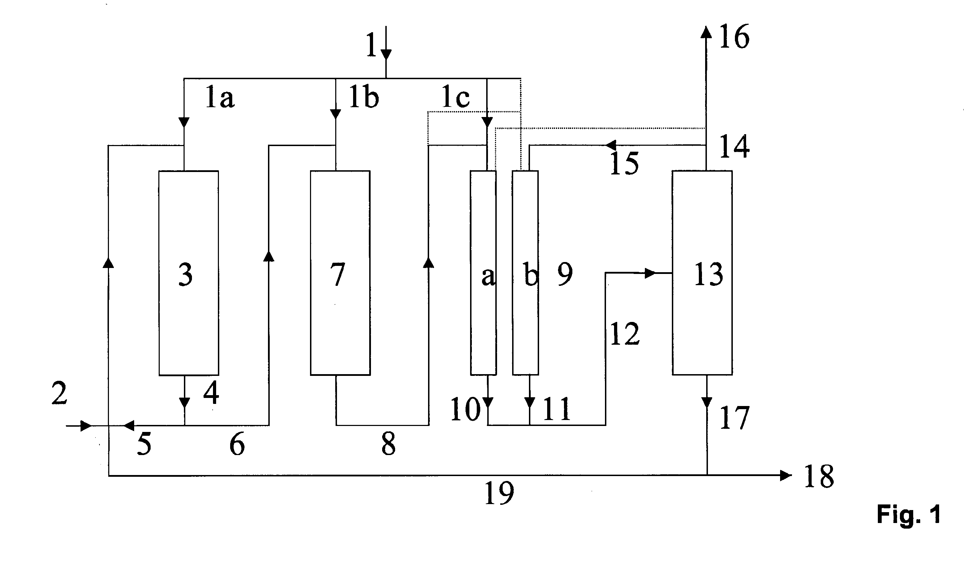

Es kann/können ein oder mehrere Reaktor(en) wechselweise mit zwei unterschiedlichen Isobuten-haltigen Gemischen beschickt werden. Wird nur ein Reaktor wechselweise mit den zwei unterschiedlichen Gemischen (I) bzw. (II) beschickt, so ist, um einen störungsfreien kontinuierlichen Betrieb zu ermöglichen, die Bereitstellung von Vorrats- oder Pufferbehältern, in denen die Gemische (I) und (II) bereitgestellt werden, notwendig. Vorzugsweise werden deshalb mindestens zwei Reaktoren oder Reaktoreinheiten im Wechsel betrieben. Werden zwei oder mehr Reaktoren wechselweise mit zwei unterschiedlichen Isobuten-haltigen Gemischen beschickt, so können diese in Serie oder parallel geschaltet sein. Die zwei oder mehr Reaktoren können dabei entweder jeweils gleichzeitig beide bzw. alle mit dem TBA-ärmeren Gemisch (II) oder TBA-reicheren Gemisch (II) beschickt werden. Zur Minimierung der Größe oder zur Vermeidung von Vorrats- bzw. Pufferbehältern ist es besonders vorteilhaft zumindest zwei parallel zueinanderverschaltete Reaktoren vorzusehen, die eine Reaktoreinheit bilden, wobei die Reaktoren der Reaktoreinheit wechselweise mit den zwei unterschiedlichen Isobuten-haltigen Gemischen beschickt werden, wobei zumindest zu Beginn eines jeden Wechseltaktes einem der parallel betriebenen Reaktoren das Gemisch (I) und dem anderen Reaktor zeitgleich das Gemisch (II) zugeführt wird. Sind mehr als zwei parallel geschaltete Reaktoren in einer Reaktoreinheit vorhanden, so ist die Verschaltung und das Umschalten von Gemisch (I) auf Gemisch (II) bzw. umgekehrt von der gewünschten Taktzeit (Zeitraum nach dem in einem Reaktor von einem Gemisch auf das andere Gemisch umgestellt wird) abhängig (siehe beispielhafte Erläuterungen zu den Varianten der Ausführungsformen gemäß Fig. 1).

Die beiden verschiedenen, durch die wechselseitige Beschickung des Reaktors bzw. der Reaktoren erhaltenen Reaktorausträge des bzw. der im Wechsel betriebenen Reaktoren können zusammen in einer Destillationseinheit in einen Kohlenwasserstoffe aufweisenden Strom, der insbesondere nicht umgesetzte Olefine und gesättigte Kohlenwasserstoffe aufweisen kann, und Roh-tert.-Butanol getrennt werden. Der bei dieser Trennung erhaltene Kohlenwasserstoff aufweisende Strom wird vorzugsweise zumindest teilweise als Wasser- und TBA-armes Gemisch (II) wieder zur Beschickung des oder der wechselseitig betriebenen Reaktoren eingesetzt. Ebenso ist es möglich, dass die beiden verschiedenen, durch die wechselseitige Beschickung erhaltenen Reaktorausträge des im Wechsel betriebenen Reaktors getrennt in zwei Destillationseinheiten in Kohlenwasserstoff aufweisende Ströme und Roh-tert.-Butanol aufgetrennt werden. In diesem Fall wird bevorzugt der als Kopfprodukt erhaltene Kohlenwasserstoffe aufweisende Strom mit dem höheren Isobutengehalt zumindest teilweise zur Beschickung des im Wechsel betriebenen Reaktors verwendet. Das als Kopfprodukt erhaltene TBA- und wasserarme Gemisch (II), welches in den wechselseitig betriebenen Reaktor zurückgeführt wird, weist vorzugsweise einen Isobutengehalt von 1 bis 30 Massen-%, bevorzugt von 3 bis 20 Massen-%, ganz besonders von 5 bis 12 Massen-% auf. Die jeweiligen Sumpffraktionen, die TBA aufweisen, sogenanntes Roh-TBA, kann einer Aufarbeitung, z. B. einer Destillation zugeführt werden. Ein Teil der Sumpffraktion kann auch dem Ausgangsstoffgemisch, mit welchem der erste Reaktor der Reaktorkaskade beschickt wird, zugegeben werden.

Wird im Nachfolgenden von einem wechselseitig beschickten Reaktor gesprochen, so sollen darunter auch mehrere Reaktoren, insbesondere Reaktoreinheiten, die zwei oder mehr parallel geschaltete Reaktoren aufweisen, verstanden werden.

Das Verfahren wird vorzugsweise so durchgeführt, dass die wechselweise Beschickung des Reaktors so erfolgt, dass der Wechsel der Gemische in einem Takt von 0,5 bis 10 Stunden erfolgt. Vorzugsweise betragen die Taktzeiten für die beiden Fahrzuständen bevorzugt von 0,5 bis 5 Stunden und besonders bevorzugt von 1 bis 2,5 Stunden. Werden Wechselzeiten von deutlich mehr als 10 Stunden überschritten, so kann es bei der Beschickung mit Wasser-armen Gemisch (II) zu einer zu großen Aktivitätssteigerung des Katalysators führen, was zu einer vermehrten Nebenproduktbildung führen kann. Bei einem deutlichen Unterschreiten der Taktzeiten von 0,5 Stunden ist der Aktivitätseffekt, der durch die Ausbildung bzw. den Abbau der Hydrathülle um den Katalysator erzielt wird, nicht mehr zu beobachten.

Das zeitliche Verhältnis der Beschickung des Reaktors (Verhältnis der Taktzeiten zueinander, also der Zeit, in der das an Wasser reichere Gemisch (I) in den Reaktor gefahren wird, zu der Zeit, in der das an Wasser und TBA arme Gemisch (II) eingespeist wird) kann gleich 1 oder größer oder kleiner 1 betragen. Vorzugsweise wird ein zeitliches Verhältnis der Beschickung des Reaktors mit an Wasser und TBA reicherem Gemisch (I) zu Beschickung mit an Wasser und TBA ärmeren Gemisch (II) von 1,5 zu 1 bis 2,5 zu 1 eingestellt. Sollen Verhältnisse der Taktzeiten unterschiedlich von 1 in einer Reaktoreinheit, bestehend aus mindestens zwei parallel geschalteten Reaktoren, in denen die beiden verschiedenen Eduktgemische I bzw. II parallel umgesetzt werden, eingestellt werden, so gibt es verschiedene Möglichkeiten dies zu realisieren, von denen beispielhaft drei nachfolgend beschrieben werden:
a) Der erste Reaktor wird für eine Zeitspanne a mit Gemisch (I) beschickt. Gleichzeitig wird der zweite Reaktor für eine Zeitspanne b mit dem Gemisch (II) beschickt und anschließend für eine Zeitspanne c mit Gemisch (I) beschickt, wobei gilt, dass die Summe der Zeitspannen b und c der Zeitspanne a entspricht. Daraus ergibt sich ein Verhältnis der Taktzeiten von (a+c)/b bei einer Gesamttaktzeit von a.
b) Werden die entsprechenden Edukte (I) und (II) vertauscht, ergibt sich entsprechend der reziproke Wert für das zeitliche Verhältnis der Beschickung.
c) Bei einer Reaktoreinheit, die mindestens drei Reaktoren beinhaltet, können beispielsweise jeweils zwei Reaktoren mit Gemisch (I) und ein Reaktor mit Gemisch (II) für eine Zeitspanne a beschickt werden. Daraus ergibt sich ein zeitliches Verhältnis der Beschickung von 2 zu 1. Nach Ende der Taktzeit wird die Beschickung so umgestellt, dass der Reaktor, der im letzten Takt mit Gemisch (II) beschickt wurde, nun mit Gemisch (I) beschickt wird. Einer der Reaktoren, die im letzten Takt mit Gemisch (I) beschickt wurden, und zwar der Reaktor, der im vorletzten Takt ebenfalls mit Gemisch (I) beschickt wurde, wird nun mit Gemisch (II) beschickt, während der andere Reaktor, der im vorletzten Takt mit Gemisch (II) beschickt wurde, noch einen Takt lang mit Gemisch (I) beschickt wird. Beim nächsten Taktwechsel wird die Beschickung wieder entsprechend geändert.

Die optimalen Bedingungen für Taktzeiten und Taktzeitverhältnis sind abhängig von der LHSV, der Temperatur und der Zusammensetzung der beiden Reaktorzuflüsse und können leicht durch Vorversuche ermittelt werden.

Es hat sich als vorteilhaft erwiesen, wenn die LHSV im Reaktors, der im Wechsel gefahren wird, auf einen Wert von 0,1 bis 5, bevorzugt von 0,4 bis 3 h⁻¹ (Liter Einsatzgemisch je Liter Katalysator je Stunde) eingestellt wird, wenn das Wasser- und TBA-reichere Gemisch (I) in den Reaktor gefahren wird. Die LHSV für das TBA- und wasserarme Kohlenwasserstoffgemisch (II), welches Isobuten aufweist, wird vorzugsweise auf einen Wert im Bereich von 0,1 bis 4,5 h⁻¹, insbesondere im Bereich von 0,3 bis 3 h⁻¹ eingestellt.

Da durch die Hydratisierung von Isobuten Wasser verbraucht wird, sinkt der Wassergehalt in der Reaktionsmischung ständig ab. Um eine möglichst hohe Ausbeute und Reaktionsgeschwindigkeit zu erhalten, ist es vorteilhaft Wasser nachzudosieren. Dies kann beispielsweise dadurch geschehen, dass in einem Rohrreaktor an verschiedenen Stellen Wasser eingespeist wird. In der Praxis ist es jedoch schwierig, genau die erforderliche Wassermenge einzubringen und zu erreichen, dass sofort eine homogene Lösung entsteht. Es ist technisch einfacher und daher vorteilhaft, mehrere Reaktoren in Reihe zu schalten und die notwendige Wassermenge jeweils vor dem Eintritt des Reaktionsgemisches in den Reaktor, also z. B. zwischen den Reaktoren einzuspeisen. Bei den Reaktoren der Reaktorkaskade, die nicht im Wechsel betrieben werden, wird vor dem Eintritt des Reaktionsgemisches in den jeweiligen Reaktor bevorzugt Wasser eingespeist. Die Zugabe von Wasser kann vor jedem Reaktor erfolgen, wobei eine Zugabe von Wasser zu dem oder den wechselseitig betriebenen Reaktoren zumindest dann nicht erfolgt, wenn diese mit dem Wasser- und TBA-armen Gemisch beschickt werden.

Die Wassermenge im Eduktgemisch stammt vorzugsweise aus einer tert.-Butanol-WasserLösung, die, abgesehen von der Anfahrphase, im Prozess selbst nach Abtrennen der Kohlenwasserstoffe, z. B. durch Destillation, anfällt, d. h. durch Rezyklieren eines Teils des Reaktoraustrags. Wenn diese Wassermenge nicht ausreicht, kann zusätzlich Wasser (Frischwasser, Reaktionswasser oder auch eine Mischung mit tert.-Butanol) eingespeist werden. Die Wassermenge im Gemisch, das einem Reaktor zugeführt wird, ist abhängig von dem Verhältnis von TBA zum C₄-Kohlenwasserstoff-Gemisch. Bevorzugt wird, mit Ausnahme des wechselseitig betriebenen Reaktors, maximal soviel Wasser eingesetzt, wie im TBA/C₄₋Kohlenwasserstoff-Gemisch löslich ist, so dass eine homogene Lösung vorliegt. Insbesondere werden, bis ggf. auf den letzten Reaktor, Wassergehalte im TBA/C₄-Kohlenwasserstoff-Gemisch eingestellt, die geringer als die jeweilige maximalen Löslichkeiten sind. Ganz besonders bevorzugt werden Wasserkonzentrationen eingestellt, wie sie in der noch nicht veröffentlichten Patentschrift DE 102 59 413.9, bzw. DE 103 30 710.9 beansprucht werden. In die Reaktoren kann wie gesagt reines Wasser oder auch eine Mischung mit tert.-Butanol eingebracht werden. Es kann auch ein Teil des durch die Umsetzung erhaltenen TBA zur Herstellung eines homogenen Gemisches mit Wasser und dem Isobuten-haltigen Kohlenwasserstoffgemisch rezykliert werden.

Die Temperaturen, bei denen das erfindungsgemäße Verfahren durchgeführt wird, betragen vorzugsweise von 30 bis 120 °C. Bei tieferen Temperaturen ist die Reaktionsgeschwindigkeit zu gering und bei höheren treten vermehrt Nebenreaktionen, wie beispielsweise die Oligomerisierung der Olefine auf. Vorzugsweise werden die Reaktoren im Temperaturbereich 35 bis 80 °C betrieben. Die Temperaturen in den verschiedenen Reaktoren können gleich oder verschieden innerhalb des angegebenen Bereichs sein. In einer bevorzugten Verfahrensvariante werden aufeinanderfolgende Reaktoren mit jeweils einer geringeren Temperatur als der vorangegangene Reaktor betrieben. Da mit sinkender Temperatur die Gleichgewichtslage günstiger wird, kann somit ein höherer Umsatz erreicht werden. Allerdings ist es nicht sinnvoll, die Temperatur unter 35 °C zu senken, da dann die Reaktion für ein technisches Verfahren zu langsam wird. Der im Wechsel betriebene Reaktor oder die im Wechsel betriebene Reaktoreinheit kann je nach Einsatzgemisch nach jedem Wechsel bei gleichen oder unterschiedlichen Temperaturen betrieben werden.

Jeder Reaktor kann adiabatisch oder quasi isotherm, d. h., mit einer Temperaturdifferenz von Reaktorzulauf zu Reaktorausgang von kleiner 10 K, bevorzugt kleiner 5 K und besonders bevorzugt kleiner 1 K betrieben werden. Ein zu hoher Temperaturanstieg ist wegen der ungünstigen Gleichgewichtsbeeinflussung (Rückspaltung) und der Zunahme von Nebenreaktionen zu vermeiden. Vorzugsweise werden alle Reaktoren der Reaktorkaskade mit einer Temperaturdifferenz des Reaktionsgemisches von Reaktoreingang zu Reaktorausgang von kleiner 10 K betrieben werden. Die Temperatur kann durch Zuführen oder Abführen von Wärme mittels geeigneter Apparaturen, z. B. Reaktoren mit als Wärmeaustauscher ausgeführten Wänden, eingestellt bzw. quasi konstant gehalten werden.

Die erfindungsgemäße Umsetzung kann bei einem Druck gleich oder über dem Dampfdruck des Einsatz-Kohlenwasserstoffgemisches bei der jeweiligen Reaktionstemperatur durchgeführt werden, vorzugsweise bei einem Druck unter 40 bar. Um in den Reaktoren Verdampfungsprobleme zu vermeiden, sollte der Druck 2 bis 4 bar höher als der Dampfdruck des Reaktionsgemisches sein.

Das Verfahren kann in chargenweise oder kontinuierlich arbeitenden Reaktoren, die üblicherweise bei Feststoff/Flüssigkeits-Kontaktreaktionen zum Einsatz gelangen, durchgeführt werden. Bei der Verwendung von kontinuierlich arbeitenden Strömungsreaktoren bedient man sich vorzugsweise, jedoch nicht zwingend, eines Festbetts. Wenn ein Festbett-Strömungsreaktor verwendet wird, kann die Flüssigkeit aufwärts oder abwärts strömen. Meistens wird ein Abwärtsströmen der Flüssigkeit bevorzugt. Der im Wechsel betriebene Reaktor bzw. die im Wechsel betriebene Reaktoreinheit wird vorzugsweise ebenfalls von oben nach unten durchströmt.

Weiterhin ist es möglich, den Reaktor unter teilweiser Produktrückführung (Schlaufenfahrweise) oder im geraden Durchgang zu betreiben. Werden einer oder mehrere der Reaktoren unter Produktrückführung betrieben, so wird vorzugsweise ein Kreislauffaktor (Verhältnis von im Kreis gepumpter Menge zu Frischzulauf) von 0,1 bis 10 eingestellt. Der Kreislauffaktor für den ersten Reaktor beträgt dabei bevorzugt 1 bis 5, insbesondere 2 bis 3,5. In einer bevorzugten Ausführungsart des erfindungsgemäßen Verfahrens kann der erste Reaktor unter Produktrückführung und die weiteren Reaktoren bzw. Reaktoreinheiten im geraden Durchgang betrieben werden, wobei insbesondere der letzte Reaktor (Reaktoreinheit) im Wechsel betrieben wird. Die Anzahl der verwendeten Reaktoren beträgt bevorzugt von 2 bis 10, ganz besonders bevorzugt von 3 bis 5 (Hierbei zählt eine Reaktoreinheit als ein Reaktor.).

Bei der Verwendung von Rohrreaktoren kann das Verhältnis von Länge zu Durchmesser der Katalysatorschüttung variiert werden, entweder durch die geometrischen Maße des Reaktors oder durch dessen Füllgrad. Bei gleicher Kontaktmenge und Belastung (LHSV) können somit unterschiedliche Leerrohrgeschwindigkeiten erreicht werden. Reaktoren, bei denen ein Teil des Reaktionsgemisches zurückgeführt wird, können z. B. mit Leerrohrgeschwindigkeiten von 12 bis 60 m/h, bevorzugt mit 12 bis 30 m/h betrieben werden. In den Reaktoren, die im geraden Durchgang durchströmt werden, können die Leerrohrgeschwindigkeiten im Bereich von 0,8 bis 55 m/h und besonders bevorzugt im Bereich von 1 bis 25 m/h liegen.

Die Katalysatorbelastung (LHSV) beträgt bei Reaktoren, die unter Produktrückführung betrieben werden, vorzugsweise von 0,3 bis 10 h⁻¹, bevorzugt von 1 bis 6 h⁻¹. Und besonders bevorzugt von 2 bis 5 h⁻¹. Bei Reaktoren, die im geraden Durchgang durchströmt werden, liegen die Belastungen vorzugsweise im Bereich von 0,1 bis 5,0 h⁻¹, bevorzugt im Bereich von 0,4 bis 3 h⁻¹ und besonders bevorzugt im Bereich von 0,6 bis 2 h⁻¹.

Das erfindungsgemäße Verfahren wird in einer Reaktorkaskade mit mindestens einem im Wechsel betriebenen Reaktor oder mindestens einer im Wechsel betriebenen Reaktoreinheit, die jeweils gleiche oder unterschiedliche Reaktortemperaturen aufweisen können, durchgeführt. Unter Reaktoreinheit versteht man in diesem Zusammenhang einen oder zwei parallel zueinanderverschalteter Reaktor(en), die wechselweise mit zwei unterschiedlichen Stoffgemischen beschickt werden können. Vorzugsweise wird das Verfahren in mehreren, hintereinandergeschalteten Reaktoren oder Reaktoreinheiten, die in Fließrichtung sinkende Temperaturen aufweisen durchgeführt.

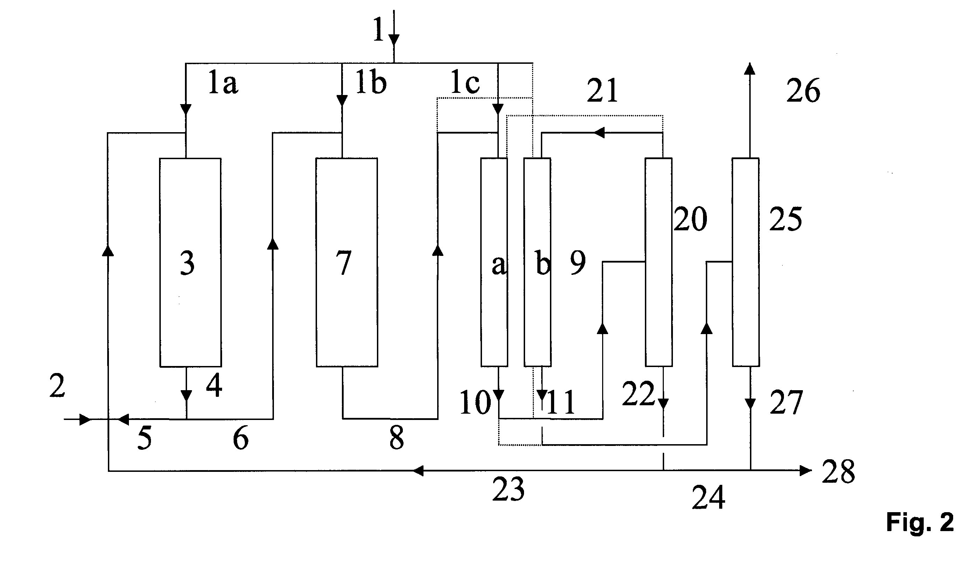

Das erfindungsgemäße Verfahren kann in verschiedenen Ausführungsformen durchgeführt werden. Zwei bevorzugte Ausführungsformen der vorliegenden Erfindung sind als Blockschema in den Figuren Fig. 1 und Fig. 2 beispielhaft dargestellt Diese Schemata weisen drei Reaktoren bzw. Reaktoreinheiten auf. Es versteht sich von selbst, dass das erfindungsgemäße Verfahren zur Herstellung von TBA auch unter Verwendung von zwei oder mehr als drei Reaktoren (bzw. Reaktoreinheiten) durchgeführt werden kann.

In der ersten Ausführungsform des erfindungsgemäßen Verfahrens gemäß Fig. 1 werden Reaktionswasser (1a), Isobuten-haltiges Einsatzkohlenwasserstoffgemisch (2), Roh-TBA (19) und ein Teil (5) des Reaktionsaustrags (4) des Reaktors (3) in den Reaktor (3) geleitet Ein Teil (6) des Austrags (4) wird zusammen mit Reaktionswasser (1b) im zweiten Reaktor (7) umgesetzt. Der Austrag (8) des Reaktors (7) wird zusammen mit Reaktionswasser (1c) entweder in den Reaktor (9a) oder nach Umschaltung in den Reaktor (9b) geleitet. Ein Teil (15) des Kopfproduktes (14) aus Kolonne (13) wird entweder in den Reaktor (9b) oder nach Umschaltung in den Reaktor (9a) eingespeist. Gegebenenfalls wird ein Teil des Wassers in Strom (15) in einer nicht dargestellten Vorrichtung entfernt. Beispielsweise kann heterogen vorliegendes Wassers mit Hilfe eines Dekanters abgetrennt werden. Die beiden Austräge (10) und (11) aus den beiden Reaktoren (9a) und (9b) werden vereinigt und als Strom (12) in die Destillationskolonne (13) gefahren und dort in ein Kopfprodukt (14), das Isobuten und andere leichtsiedende Kohlenwasserstoffe enthält, und in Roh-TBA (17) getrennt. Ein Teil (16) des Kopfproduktes (14) wird zur Ausschleusung von Kohlenwasserstoffen abgetrennt. Ein Teil (19) des Roh-TBA (17) wird in den Reaktor (3) zurückgefahren. Der andere Teil (18) kann in einer nicht dargestellten Anlage in Rein-TBA und/oder TBA/Wasser-Azeotrop aufgearbeitet werden. Die Aufarbeitung kann beispielsweise durch Destillation oder Extraktion erfolgen. Zur Vergleichmäßigung der Zusammensetzung des Stromes (12) kann es vorteilhaft sein, in diesem Strom einen Pufferbehälter (nicht abgebildet) vorzusehen, aus dem die gemischten Austräge aus den Reaktoren in die Destillationskolonne (13) gefahren werden.

In der Ausführungsform des erfindungsgemäßen Verfahren gemäß Fig. 1 können in einer Variante die beiden Reaktoren (9a) und (9b) durch einen einzigen Reaktor (9) ersetzt werden. Dieser wird wechselweise entweder mit einem Gemisch aus Strom (8) und Reaktionswasser oder mit dem Destillat (15) beschickt. Bei dieser Variante nach Fig. 1 sind, um einen störungsfreien kontinuierlichen Betrieb zu ermöglichen, zusätzliche Pufferbehälter (in Figur 1 nicht dargestellt) für die Ströme (8), (10), (11) und (15) notwendig. Um ein Taktverhältnis abweichend von 1 einstellen zu können muss ebenfalls entweder gemäß dieser Variante gefahren werden oder es kann eine weitere Variante zur Anlage gemäß Fig. 1 vorgesehen werden, bei der noch ein dritter Reaktor 9c vorhanden ist. Durch jeweiliges weiterschalten kann ein Taktverhältnis von 2 zu 1 eingestellt werden, in dem zunächst in die Reaktoren 9a und 9b Wasser und Gemisch aus Strom 8 gefahren wird und zeitgleich in Reaktor 9c Destillat 15 gefahren wird. Nach Ablauf der Taktzeit wird das Destillat nun auf den Reaktor 9b gefahren, während die Reaktoren 9a und 9c während dieses Taktes mit Wasser und dem Gemisch aus Strom 8 beschickt werden. Nach Ablauf der Taktzeit wird wiederum umgestellt, so dass nun auf den Reaktor 9a das Destillat gefahren wird, während die anderen Reaktoren mit Wasser und Strom 8 versorgt werden. Nach Ende dieser Taktzeit wird wieder gemäß dem ersten Takt verschaltet. Um weitere von 1 verschiedene Taktverhältnisse einzustellen, kann, unabhängig davon ob zwei oder mehr Reaktoren vorhanden sind, auch vor Ende des eigentlichen Taktes zumindest ein Reaktor bereits auf das andere Edukt umgestellt werden. Auf diese Weise sind sämtliche Verhältnisse einstellbar. Die gerade beschriebenen Möglichkeiten zur Variation des Taktverhältnisses bei zwei oder mehr vorhandenen Reaktoren sind nicht nur auf Anlagen gemäß Fig. 1 bzw. deren Varianten beschränkt sondern können generell im erfindungsgemäßen Verfahren zur Anwendung kommen.

In der zweiten Ausführungsform des erfindungsgemäßen Verfahrens gemäß Figur 2 werden Reaktionswasser (1a), Isobuten-haltiges Einsatzkohlenwasserstoffgemisch (2), Roh-TBA (23) und ein Teil (5) des Reaktionsaustrags (4) des Reaktors (3) in den Reaktor (3) geleitet. Ein Teil (6) des Austrags (4) wird zusammen mit Reaktionswasser (1b) im zweiten Reaktor (7) umgesetzt. Der Austrag (8) des Reaktors (7) wird zusammen mit Reaktionswasser (1c) entweder in den Reaktor (9a) oder nach Umschaltung in den Reaktor (9b) geleitet. Das Kopfproduktes (21) aus Kolonne (20) wird entweder in den Reaktor (9b) oder nach Umschaltung in den Reaktor (9a) eingespeist. Der Reaktoraustrag (10) oder (11) wird, wenn er durch Umsetzung von Strom (8) mit Reaktionswasser (1c) entstanden ist, in die Kolonne (20) geleitet. Der Reaktionsaustrag (10) oder (11) wird, wenn er durch Umsetzung des Destillats (21) der Kolonne (20) entstanden ist, in die Kolonne (25) gefahren. In der Kolonne (20) wird der Austrag (10) oder (11) in ein Isobuten-haltiges Kohlenwasserstoffgemisch, das, gegebenenfalls nach Entfernung von Wasser in einer nicht dargestellten Vorrichtung, beispielsweise eines Dekanters zur Abtrennung von heterogen vorliegenden Wasser, in den Reaktor (9a) oder (9b) zurückgeführt wird, und Roh-TBA (22) getrennt. In Kolonne (25) wird der Austrag (10) oder (11) in ein Kopfprodukt (26), das weniger Isobuten als das Destillat (21) enthält, und in Roh-TBA (27) getrennt. Ein Teil (23) des erzeugten Roh-TBA (Strom (22) oder Strom (27) oder ein Gemisch aus Strom (22) und Strom (27)) wird in den Reaktor (3) zurückgefahren. Der andere Teil (28) kann in einer nicht dargestellten Anlage in Rein-TBA und/oder TBA/Wasser-Azeotrop aufgearbeitet werden. Der Strom (26) wird ausgeschleust und kann in anderen Anlagen nach bekannten Verfahren, z. B. durch Destillation aufgearbeitet werden.

Bei Ausführungsform 2, bei der die beiden unterschiedlichen Austräge des im Wechsel betriebenen Reaktor oder der im Wechsel betriebenen Reaktoreinheit, in zwei verschiedenen Destillationseinheiten aufgearbeitet werden, ist es zweckmäßig, nach Umschaltung der Reaktorzuläufe die Umschaltung der Reaktorausträge auf die beiden Destillationseinheiten mit einer gewissen Zeitverzögerung vorzunehmen. Die optimale Größe der Zeitdifferenz zwischen der Umschaltung der Zuläufe und der Abläufe ist unter Anderem von der Reaktorbelastung (LHSV) abhängig und lässt sich leicht durch Verfolgung der Zusammensetzung der Reaktorausträge bestimmen. Vorzugsweise erfolgt die Umschaltung der Abläufe deshalb automatisch oder halbautomatisch und zwar entweder zeitgesteuert in Abhängigkeit von der Umschaltung der Zuläufe oder gesteuert über eine Online-Messung der Zusammensetzung des Ablaufs bzw. der Online-Messung der Konzentration einer Komponente, insbesondere der Konzentration von Wasser oder TBA.

Eine wie gerade beschriebene verzögerte Umschaltung der Zu- und Abläufe ist nicht beschränkt auf die in Fig. 2 beschriebene Ausführungsform sondern kann allgemein immer dann vorgesehen werden, wenn die Reaktorausträge aus den wechselseitig betriebenen Reaktoren in zwei verschiedene Destillationskolonnen zur Auftrennung überführt werden.

Bei einem Verfahren nach Figur 2 ist es zur Erleichterung des kontinuierlichen Betriebs zweckmäßig, für die Zuläufe der Kolonnen (20) und (25) Pufferbehälter vorzusehen.

Es sei darauf hingewiesen, dass auch bei dieser zweiten Ausführungsform analog wie bei der Ausführungsform gemäß Fig. 1, die Reaktoren (9a) und (9b) in einer Variante durch einen einzigen Reaktor ersetzt werden können oder durch einen oder mehrere weitere(n) Reaktor(en) ergänzt werden können.

Die Ausführungsform des erfindungsgemäßen Verfahrens gemäß Fig. 2 unterscheidet sich von der Ausführungsform gemäß Fig. 1 im Wesentlichen dadurch, dass die Ausführungsform gemäß Fig. 2 eine Destillationsstufe mehr aufweist und die beiden verschiedenen Produktausträge des/der im Wechsel betriebenen Reaktors/Reaktoren in zwei verschiedenen Kolonnen aufgetrennt werden. Dadurch kann mit einem Verfahren nach der Ausführungsform gemäß Fig. 2 ein höherer Isobuten-Umsatz erreicht werden, als mit einem Verfahren gemäß Fig. 1. Allerdings erfordert ein Verfahren nach der Ausführungsform gemäß Fig. 2 einen höheren Kapitaleinsatz und höhere Betriebskosten.

Optional kann, wenn ein noch höherer Isobuten-Umsatz zu TBA als in einem Verfahren nach der Ausführungsform gemäß Fig. 2 gewünscht wird, das Isobuten-haltige Destillat (26 in Fig. 2) zur wechselweise Beschickung eines weiteren Reaktor (beispielsweise Reaktor 7 in Fig. 2) eingesetzt und das daraus entstandene Produkt in einer dritten Kolonne (in Fig. 2 nicht dargestellt) getrennt werden.

### Beispiele

Die Versuche wurden in einem Rohrreaktor mit einem inneren Durchmesser von 2,1 cm und einer Länge von 40 cm durchgeführt. Dieser enthielt bei allen Versuchen 120 ml Ionenaustauscherharz Amberlyst® 35 Wet in der H-Form. Bei allen Versuchen wurde der Reaktor von oben nach unten durchströmt. Der Reaktor wurde jeweils isotherm bei 55 °C und einem Reaktionsdruck von 15 bar betrieben, sodass das Einsatz- bzw. Produktgemisch fast ausschließlich in der flüssigen Phase vorlag.

Für die Versuche wurde ein an Isobuten armer Raffinatstrom (X) mit folgender Zusammensetzung verwendet:
n-Butan 15,4 Massen-%
Isobutan 1,4 Massen-%
1-Buten 31,3 Massen-%
2-Buten (cis und trans) 46,6 Massen-%
Isobuten Massen-5,3%
Zur besseren Übersichtlichkeit wurden im folgenden Text die C₄-Kohlenwasserstoffe, die nicht Isobuten sind, als Rest-C₄ ausgewiesen. Aus dem Gemisch (X = Xa) wurde durch Zumischen von TBA und Wasser das Einsatzgemisch (Xb) hergestellt. Durch Mischen von Gemisch (Xa) mit Gemisch (Xb) im Verhältnis von 47 zu 61,6 entstand das Einsatzgemisch (Xc)

**Tabelle 1: Zusammensetzung der Einsatzgemische.**

| Bezeichnung | Zusammensetzung in Massen-% |
|---|---|
| Xa (homogen) | 5,32 Isobuten |
| | 94,68 Rest-C₄ |
| Xb | 4,06 Isobuten |
| | 72,27 Rest-C₄ |
| | 17,74 TBA |
| | 5,60 Wasser |
| | 0,334 Sonstige Stoffe |
| Xc | 4,60 Isobuten |
| | 81,95 Rest-C₄ |
| | 10,06 TBA |
| | 3,18 Wasser |
| | 0,21 sonstige Stoffe |

Die Analyse der Zusammensetzung von Einsatz- und/oder Produktstoffgemischen erfolgte gaschromatographisch auf einer DBI-Säule (Agilent J&W). Da die verschiedenen C₄-Komponenten auf der DBI-Säule nicht trennbar sind, wurde die Aufteilung der C₄-Komponente auf einer Al₂O₃/Na₂SO₄-Säule der Fa. Chrompack analysiert.

### Beispiel 1 (erfindungsgemäß):

Der Reaktor wurde wechselweise mir den Gemischen (Xa) und (Xb) beschickt. Der Zeittakt betrug 4 Stunden. Es wurde also in den ersten vier Stunden das Gemisch (Xa) eingespeist, in den nächsten vier Stunden das Gemisch (Xb), in den folgenden vier Stunden wiederum das Gemisch (Xa) und so weiter. Die Zulaufgeschwindigkeit betrug für das Gemisch (Xa) 47 g/h und für das Gemisch (Xb) 61,6 g/h. Das Gemisch wurde 48 h lang (6 Taktzeiten von Gemisch (Xa) und 6 Taktzeiten von Gemisch (Xb)) gesammelt und danach von diesem Gemisch eine Durchschnittsanalyse erstellt.

Die heterogene Probe hatte folgende Zusammensetzung (in Massen-%):
4,19 Isobuten
81,95 Rest-C₄
10,62 TBA
3,04 Wasser
0,2 sonstige Stoffe

Daraus berechnete sich ein Isobuten-Umsatz von 9,2 %.

### Beispiel 2 (Vergleichsbeispiel):

In den Reaktor wurde das Einsatzgemisch (Xc) eingespeist. Die Zuleitungsgeschwindigkeit betrug 54,3 g/h. Der Reaktionsaustrag von 48 h wurde gesammelt und eine Durchschnittsanalyse erstellt.

Der Reaktionsaustrag hatte folgende Zusammensetzung (in Massen-%):
4,40 Isobuten
81,95 Rest-C₄
10,33 TBA
3,11 Wasser
0,21 sonstige Stoffe

Daraus berechnete sich ein Isobuten-Umsatz von 4,6 %.

Bei den beiden Versuchen wurden in 48 h von jeder Komponente die gleiche Stoffmenge in den Reaktor gefahren. Dennoch ergab der Versuch in Beispiel 1 gemäß der Erfindung einen doppelt so großen Isobuten-Umsatz wie der Versuch im Vergleichsbeispiel (Beispiel 2).

## Patentansprüche

1. Verfahren zur Herstellung von tert.-Butanol (TBA) durch Umsetzung eines Isobuten-haltigen Kohlenwasserstoffgemisches mit Wasser an einem festen sauren Katalysator in einer Reaktorkaskade,
**dadurch gekennzeichnet,**
**dass** mindestens ein Reaktor wechselweise mit zwei verschiedenen Isobuten-haltigen Kohlenwasserstoffgemischen beschickt wird, wobei eines der Gemische sowohl einen höheren tert.-Butanol-Gehalt als auch einen höheren Wassergehalt als das andere Gemisch aufweist.

2. Verfahren nach Anspruch 1,
**dadurch gekennzeichnet,**
**dass** das Kohlenwasserstoffgemisch mit dem geringeren tert.-Butanol-Gehalt weniger als 1 Massen-% tert.-Butanol enthält.

3. Verfahren nach Anspruch 2,
**dadurch gekennzeichnet,**
**dass** das Kohlenwasserstoffgemisch mit dem geringeren tert.-Butanol-Gehalt weniger als 0,1 Massen-% tert.-Butanol enthält.

4. Verfahren nach Anspruch 1,
**dadurch gekennzeichnet,**
**dass** das Kohlenwasserstoffgemisch mit dem geringeren tert.-Butanol-Gehalt einen Gehalt an Isobuten von 1 bis 30 Massen-% aufweist.

5. Verfahren nach einem der Ansprüche 1 bis 5,
**dadurch gekennzeichnet,**
**dass** das Kohlenwasserstoffgemisch mit dem geringeren tert.-Butanol-Gehalt einen Gehalt an Wasser aufweist, der maximal 10 % über dem Gehalt liegt, der in dem Kohlenwasserstoffgemisch löslich ist.

6. Verfahren nach einem der Ansprüche 1 bis 6,
**dadurch gekennzeichnet.**
**dass** ein Reaktor wechselweise mit zwei unterschiedlichen Isobuten-haltigen Gemischen beschickt wird.

7. Verfahren nach einem der Ansprüche 1 bis 5,
**dadurch gekennzeichnet,**
**dass** zwei parallel zueinanderverschaltete Reaktoren, die eine Reaktoreinheit bilden, wechselweise mit den zwei unterschiedlichen Isobuten-haltigen Gemischen beschickt werden.

8. Verfahren nach einem der Ansprüche 1 bis 7,
**dadurch gekennzeichnet,**
**dass** die beiden verschiedenen, durch die wechselseitige Beschickung erhaltenen Reaktorausträge des im Wechsel betriebenen Reaktors zusammen in einer Destillationseinheit in einen Kohlenwasserstoffe aufweisenden Strom und Roh-tert.-Butanol getrennt werden.

9. Verfahren nach einem der Ansprüche 1 bis 7,
**dadurch gekennzeichnet,**
**dass** die beiden verschiedenen, durch die wechselseitige Beschickung erhaltenen Reaktorausträge des im Wechsel betriebenen Reaktors getrennt in zwei Destillationseinheiten in Kohlenwasserstoff aufweisende Ströme und Roh-tert.-Butanol aufgetrennt werden und dass Kopfprodukt mit dem höheren Isobutengehalt zur Beschickung des im Wechsel betriebenen Reaktors verwendet wird.

10. Verfahren nach einem der Ansprüche 1 bis 9,
**dadurch gekennzeichnet,**
**dass** mindestens zwei Reaktoren oder Reaktoreinheiten im Wechsel betrieben werden.

11. Verfahren nach einem der Ansprüche 1 bis 10,
**dadurch gekennzeichnet,**
**dass** die wechselweise Beschickung des Reaktors so erfolgt, dass der Wechsel der Gemische in einem Takt von 0,5 bis 10 Stunden erfolgt.

12. Verfahren nach einem der Ansprüche 1 bis 11,
**dadurch gekennzeichnet,**
**dass** das zeitliche Verhältnis der Beschickung des Reaktors mit an Wasser und TBA reicherem Gemisch zu Beschickung mit an Wasser und TBA ärmeren Gemisch gleich oder größer 1 beträgt.

13. Verfahren nach Anspruch 12,
**dadurch gekennzeichnet,**
**dass** ein zeitliches Verhältnis der Beschickung des Reaktors mit an Wasser und TBA reicherem Gemisch zu Beschickung mit an Wasser und TBA ärmeren Gemisch von 1,5 zu 1 bis 2,5 zu 1 eingestellt wird.

14. Verfahren nach einem der Ansprüche 1 bis 13,
**dadurch gekennzeichnet,**
**dass** das Verfahren bei einer Temperatur von 30 bis 120 °C durchgeführt wird.

15. Verfahren nach einem der Ansprüche 1 bis 14,
**dadurch gekennzeichnet,**
**dass** in den ersten Reaktor der Reaktorkaskade ein Kohlenwasserstoffgemisch gefahren wird, welches einen Gehalt an Isobuten von größer 25 Massen-% aufweist.

16. Verfahren nach einem der Ansprüche 1 bis 15,
**dadurch gekennzeichnet,**
**dass** bei den Reaktoren der Reaktorkaskade, die nicht im Wechsel betrieben werden, vor dem Eintritt des Reaktionsgemisches in den jeweiligen Reaktor Wasser eingespeist wird.

17. Verfahren nach einem der Ansprüche 1 bis 16,
**dadurch gekennzeichnet,**
**dass** alle Reaktoren der Reaktorkaskade mit einer Temperaturdifferenz des Reaktionsgemisches von Reaktoreingang zu Reaktorausgang von kleiner 10 K betrieben werden.

18. Verfahren nach einem der Ansprüche 1 bis 17,
**dadurch gekennzeichnet,**
**dass** als Katalysator in Reaktoren, die mit einer Lineargeschwindigkeit von 1 bis 60 m/h betrieben werden, ein Ionenaustauscherharz verwendet wird, das eine mittlere Korngröße von 0,1 bis 5 mm aufweist.

19. Verfahren nach einem der Ansprüche 1 bis 18,
**dadurch gekennzeichnet,**
**dass** der Reaktor, der wechselweise mit zwei verschiedenen Isobuten-haltigen Kohlenwasserstoffgemischen beschickt wird, wobei eines der Gemische sowohl einen höheren tert.-Butanol-Gehalt als auch einen höheren Wassergehalt als das andere Gemisch aufweist, der letzte Reaktor oder eine letzte Reaktoreinheit aus zwei oder mehr parallel geschalteten Reaktoren der Reaktorkaskade ist.

## Claims

1. Process for preparing tert-butanol (TBA) by reaction of an isobutene-containing hydrocarbon mixture with water over a solid acid catalyst in a reactor cascade, **characterized in that** at least one reactor is supplied alternately with two different isobutene-containing hydrocarbon mixtures, of which one has both a higher tert-butanol content and a higher water content than the other mixture.

2. Process according to Claim 1, **characterized in that** the hydrocarbon mixture having the lower tert-butanol content contains less than 1% by mass of tert-butanol.

3. Process according to Claim 2, **characterized in that** the hydrocarbon mixture having the lower tert-butanol content contains less than 0.1% by mass of tert-butanol.

4. Process according to Claim 1, **characterized in that** the hydrocarbon mixture having the lower tert-butanol content has an isobutene content of from 1 to 30% by mass.

5. Process according to any of Claims 1 to 5, **characterized in that** the hydrocarbon mixture having the lower tert-butanol content has a water content which is not more than 10% above the content which is soluble in the hydrocarbon mixture.

6. Process according to any of Claims 1 to 5, **characterized in that** one reactor is supplied alternately with two different isobutene-containing mixtures.

7. Process according to any of Claims 1 to 5, **characterized in that** two reactors which are connected in parallel to form a reactor unit are supplied with the two different isobutene-containing mixtures.

8. Process according to any of Claims 1 to 7, **characterized in that** the two different outputs obtained from the alternately operated reactors as a result of the alternate supply of different feeds to the reactor are together separated in one distillation unit into a hydrocarbon-containing stream and crude tert-butanol.

9. Process according to any of Claims 1 to 7, **characterized in that** the two different outputs obtained from the alternately operated reactor as a result of the alternate supply of different feeds to the reactor are fractionated separately in two distillation units to give hydrocarbon-containing streams and crude tert-butanol and the overhead product having the higher isobutene content is used for supplying the alternately operated reactor.

10. Process according to any of Claims 1 to 9, **characterized in that** at least two reactors or reactor units are operated alternately.

11. Process according to any of Claims 1 to 10, **characterized in that** the alternating supply to the reactor is effected so that the mixtures are changed in a cycle of from 0.5 to 10 hours.

12. Process according to any of Claims 1 to 11, **characterized in that** the time ratio for the supply of the mixture which is relatively rich in water and TBA to the reactor to the supply of the mixture which is relatively low in water and TBA is equal to or greater than 1.

13. Process according to Claim 12, **characterized in that** a time ratio for the supply of the mixture which is relatively rich in water and TBA to the reactor to supply of the mixture which is relatively low in water and TBA of from 1.5 : 1 to 2.5 : 1 is set.

14. Process according to any of Claims 1 to 13, **characterized in that** the process is carried out at a temperature of from 30 to 120°C.

15. Process according to any of Claims 1 to 14, **characterized in that** a hydrocarbon mixture having an isobutene content of greater than 25% by mass is fed into the first reactor of the reactor cascade.

16. Process according to any of Claims 1 to 15, **characterized in that**, in the case of reactors of the reactor cascade which are not operated alternately, water is fed in before the reaction mixture enters the respective reactor.

17. Process according to any of Claims 1 to 16, **characterized in that** all reactors of the reactor cascade are operated with a temperature difference of the reaction mixture from reactor inlet to reactor outlet of less than 10 K.

18. Process according to any of Claims 1 to 17, **characterized in that** the catalyst used in reactors operated at a linear velocity of from 1 to 60 m/h is an ion-exchange resin having a mean particle size of from 0.1 to 5 mm.

19. Process according to any of Claims 1 to 18, **characterized in that** the reactor which is supplied alternately with two different isobutene-containing hydrocarbon mixtures, of which one has both a higher tert-butanol content and a higher water content than the other mixture, is the last reactor or a last reactor unit comprising two or more reactors connected in parallel of the reactor cascade.

## Revendications

1. Procédé pour la préparation de tert-butanol (TBA) par transformation d'un mélange d'hydrocarbures contenant de l'isobutène avec de l'eau sur un catalyseur acide solide dans une cascade de réacteurs, **caractérisé en ce qu'**au moins un réacteur est chargé alternativement en deux mélanges d'hydrocarbures contenant de l'isobutène différents, un des mélanges présentant une teneur en tert-butanol supérieure ainsi qu'une teneur en eau supérieure à celles de l'autre mélange.

2. Procédé selon la revendication 1, **caractérisé en ce que** le mélange d'hydrocarbures présentant la teneur en tert-butanol plus faible contient moins de 1% en masse de tert-butanol.

3. Procédé selon la revendication 2, **caractérisé en ce que** le mélange d'hydrocarbures présentant la teneur en tert-butanol plus faible contient moins de 0,1% en masse de tert-butanol.

4. Procédé selon la revendication 1, **caractérisé en ce que** le mélange d'hydrocarbures présentant la teneur en tert-butanol plus faible présente une teneur en isobutène de 1 à 30% en masse.

5. Procédé selon l'une quelconque des revendications 1 à 4, **caractérisé en ce que** le mélange d'hydrocarbures présentant la teneur en tert-butanol plus faible présente une teneur en eau qui est au maximum supérieure de 10% à la teneur qui est soluble dans le mélange d'hydrocarbures.

6. Procédé selon l'une quelconque des revendications 1 à 5, **caractérisé en ce qu'**un réacteur est chargé alternativement en deux mélanges contenant de l'isobutène différents.

7. Procédé selon l'une quelconque des revendications 1 à 5, **caractérisé en ce que** deux réacteurs commutés en parallèle l'un par rapport à l'autre, qui forment une unité de réacteur, sont chargés alternativement en deux mélanges différents contenant de l'isobutène.

8. Procédé selon l'une quelconque des revendications 1 à 7, **caractérisé en ce que** les deux produits différents évacués du réacteur, obtenus par la charge alternative, du réacteur exploité en alternance sont séparés ensemble dans une unité de distillation en un flux présentant des hydrocarbures et en tert-butanol brut.

9. Procédé selon l'une quelconque des revendications 1 à 7, **caractérisé en ce que** les deux produits différents évacués du réacteur, obtenus par la charge alternative, du réacteur exploité en alternance sont séparés séparément dans deux unités de distillation en flux présentant des hydrocarbures et en tert-butanol brut et **en ce que** le produit de tête présentant la teneur en isobutène la plus élevée est utilisé pour charger le réacteur exploité en alternance.

10. Procédé selon l'une quelconque des revendications 1 à 9, **caractérisé en ce qu'**au moins deux réacteurs ou unités de réacteur sont exploités en alternance.

11. Procédé selon l'une quelconque des revendications 1 à 10, **caractérisé en ce que** la charge alternative du réacteur a lieu de manière telle que le changement des mélanges a lieu dans un cycle de 0,5 à 10 heures.

12. Procédé selon l'une quelconque des revendications 1 à 11, **caractérisé en ce que** le rapport temporel de la charge du réacteur en mélange plus riche en eau et en TBA à la charge en mélange plus pauvre en eau et en TBA est égal ou supérieur à 1.

13. Procédé selon la revendication 12, **caractérisé en ce qu'**on règle un rapport temporel de la charge du réacteur en mélange plus riche en eau et en TBA à la charge en mélange plus pauvre en eau et en TBA de 1,5:1 à 2,5:1.

14. Procédé selon l'une quelconque des revendications 1 à 13, **caractérisé en ce que** le procédé est réalisé à une température de 30 à 120°C.

15. Procédé selon l'une quelconque des revendications 1 à 14, **caractérisé en ce qu'**un mélange d'hydrocarbures qui présente une teneur en isobutène supérieure à 25% en masse est introduit dans le premier réacteur de la cascade de réacteurs.

16. Procédé selon l'une quelconque des revendications 1 à 15, **caractérisé en ce qu'**on injecte, dans les réacteurs de la cascade de réacteurs qui ne sont pas exploités en alternance, de l'eau avant l'entrée du mélange réactionnel dans le réacteur concerné.

17. Procédé selon l'une quelconque des revendications 1 à 16, **caractérisé en ce que** tous les réacteurs de la cascade de réacteurs sont exploités à une différence de température du mélange réactionnel de l'entrée du réacteur à la sortie du réacteur inférieure à 10 K.

18. Procédé selon l'une quelconque des revendications 1 à 17, **caractérisé en ce qu'**on utilise, comme catalyseur dans les réacteurs qui sont exploités à une vitesse linéaire de 1 à 60 m/h, une résine échangeuse d'ions qui présente une grosseur moyenne des particules de 0,1 à 5 mm.

19. Procédé selon l'une quelconque des revendications 1 à 18, **caractérisé en ce que** le réacteur, qui est chargé alternativement en deux mélanges d'hydrocarbures contenant de l'isobutène différents, un des mélanges présentant une teneur en tert-butanol supérieure ainsi qu'une teneur en eau supérieure à celles de l'autre mélange, est le dernier réacteur ou une dernière unité de réacteur de deux réacteurs commutés en parallèle ou plus de la cascade de réacteurs.
